(19) Europäisches Patentamt
European Patent Office
Office européen des brevets

(11) Numéro de publication: **0 070 995**
**B1**

# (12) FASCICULE DE BREVET EUROPEEN

(45) Date de publication du fascicule du brevet: **21.11.84**

(21) Numéro de dépôt: **82104701.6**

(22) Date de dépôt: **28.05.82**

(51) Int. Cl.³: **C 07 C 49/557**, C 07 C 45/00, C 11 B 9/00, A 61 K 7/46, A 23 L 1/226

(54) **Procédé pour la préparation de cétones cycloaliphatiques polyinsaturées.**

(30) Priorité: **23.07.81 CH 4809/81**

(43) Date de publication de la demande: **09.02.83 Bulletin 83/6**

(45) Mention de la délivrance du brevet: **21.11.84 Bulletin 84/47**

(84) Etats contractants désignés: **CH DE FR GB LI NL**

(56) Documents cités:
**DE - A - 2 305 140**
**DE - A - 2 646 322**
**US - A - 3 931 326**

**Chemical Abstracts vol. 84, no. 11 15 mars 1976 Columbus, Ohio, USA I. TANAKA et al. "Allyl derivatives as cockroach repellents" pahe 118, colonne 2, abstract no. 70350x et Chemical Substance Index Vol. 84, 1976 page 2413CS, colonne 1, lignes 94 à 97**

(73) Titulaire: **FIRMENICH SA, 1, route des Jeunes, CH-1211 Genève 8 (CH)**

(72) Inventeur: **Schulte-Elte, Karl-Heinrich, 44, chemin de Carabot, CH-1213 Onex (CH)**
Inventeur: **Snowden, Roger, 5, chemin des Palettes, CH-1212 Grand-Lancy (CH)**
Inventeur: **Müller, Bernard, 16, route de Malagnon, CH-1208 Genève (CH)**

(74) Mandataire: **Salvadori, Giuseppe, Dr., c/o Firmenich S.A. Case Postale 239, CH-1211 Genève 8 (CH)**

EP 0 070 995 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Depuis leur découverte, les cétones cycloaliphatiques connues sous le nom de damascones et damascénones, notamment l'$\alpha$- et $\beta$-damascone ainsi que la $\beta$-damascénone [voir E. Demole et al., Helv. Chim. Acta, 53, 541 (1970) et le brevet suisse no. 520 479], ont trouvé une utilisation de plus en plus étendue dans tous les domaines de la parfumerie et des arômes. L'intérêt porté à ces composés est montré par le nombre croissant de publications scientifiques et brevets parus au cours de la dernière décennie et traitant de leur emploi, leur identification dans différentes sources naturelles ou tout simplement leur synthèse. En considération de l'intérêt commercial que ces composés présentent, l'industrie s'est attelée à développer des procédés de synthèse variés, procédés destinés en premier lieu à rendre leur préparation plus économique.

La présente invention a précisément trait à un procédé nouveau pour la préparation de cétones cycloaliphatiques polyinsaturées de formule

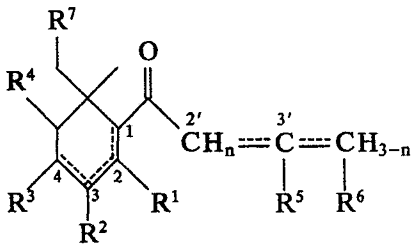

(I)

possédant une double liaison en position 2'— ou 3'- de la chaine latérale, dans laquelle l'indice n vaut 1 ou 2, et possédant en outre une double liaison isolée dans les positions 1 ou 2, ou deux doubles liaisons conjuguées dans les positions 1 et 3, comme indiqué par les pointillés et dans laquelle chacun des symboles $R^1$ à $R^7$, différents ou identiques, sert à désigner un atome d'hydrogène ou un radical alkyle inférieur, le méthyle et l'éthyle et l'éthyle de préférence, ledit procédé étant caractérisé en ce qu'on traite au moyen d'une base forte, et dans un solvant inerte, un carbinol diallylique de formule

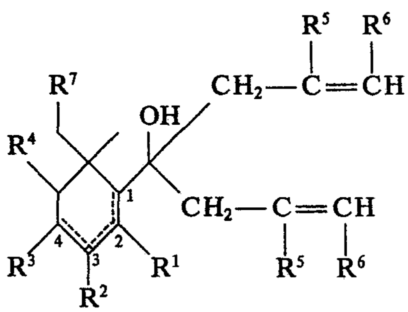

(II)

dans laquelle les symboles $R^1$ à $R^7$ ainsi que les pointillés ont le sens indiqué plus haut.

Les composés de formule (II), utilisés comme produits de départ pour le procédé de l'invention, peuvent être préparés par traitement d'un composé de formule

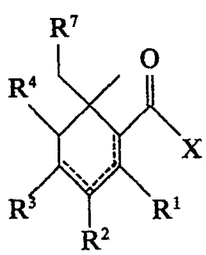

(III)

dans laquelle les symboles $R^1$ à $R^4$ et $R^7$ ainsi que les pointillés ont le sens indiqué plus hat, et le symbole X représente un groupe tel que halogène, O—CO-alkyle, O—CO-aryle, O-alkyle, O-aryle ou OH, au moyen d'un composé organo-métallique de formule

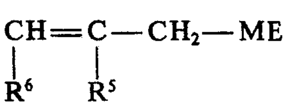

(IV)

dans laquelle le symbole ME représente une fonction métallique telle le Zn, Cd ou Mg-halogène.

Comme composé de formule (III), on utilise de préférence un ester dérivant d'un alcool aliphatique inférieur et, comme composé organométallique, un composé obtenu par réaction du chlorure ou bromure d'allyle sur le magnésium. La réaction peut s'effectuer selon les conditions usuelles d'une réaction de type de Grignard [voir à ce sujet: Helv. Chim. Acta, 54, 1767 (1971)]. Les composés de formule (III) peuvent être obtenus selon des méthodes connues et notamment décrites dans le brevet suisse no. 549 951 ou dans le brevet suisse no. 563 951, ou selon des méthodes analogues.

La réaction, qui caractérise le procédé de l'invention, consiste formellement en un clivage anionique, lequel est effectué par l'action d'une base forte. A cet effet, on peut utiliser des bases minérales ou organiques telles que les hydrures, les alkoxydes ou les hydroxydes d'un métal alcalin, le sodium et le potassium de préférence. Parmi lesdites bases, il convient de citer tout particulièrement l'hydrure de sodium ou potassium, le tert-butylate de sodium ou potassium, le tert-amylate de sodium ou le méthylate et l'éthylate de sodium.

Le choix particulier, parmi les bases appartenant à la classe définie ci-dessus, est déterminé par des considérations d'ordre économique, de sécurité et d'hygiène du travail. C'est ainsi que les alkoxydes sont préférés aux hydrures, le tert-butylate de potassium ou sodium étant employé de préférence.

On a pu déterminer que la proportion de la base utilisée doit être égale ou supérieure à la quantité stoechïométrique requise. En réalité, les rendements les meilleurs ont été obtenus en utilisant la base en excès.

Les temps de réaction observés observés sont relativement courts. A cet effet, il faut remarquer que la transformation des carbinols allyliques en leurs damascones ou damascénones correspondantes de formule (I), suivant le procédé de la présente invention, passe à travers la formation d'un énolate de formule

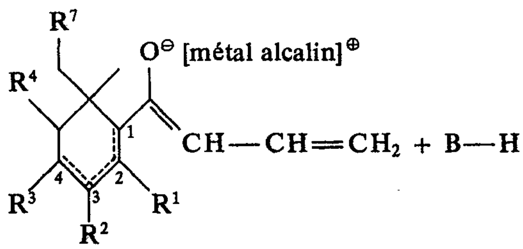

B = Base

lequel s'est révélé instable dans un milieu protique, d'où la nécessité d'arrêter rapidement la réaction lorsque celle-ci est effectuée au moyen d'un alkoxyde. C'est ainsi qu'à des températures comprises entre environ 20° et 70° C, les temps de réaction peuvent être de l'ordre de quelques minutes lorsque la base utilisée est le tert-butylate de sodium ou potassium. Par l'emploi d'hydrure de sodium, le temps de réaction est par contre plus long, 15 heures étant par exemple nécessaire pour transformer à l'aide d'hydrure de potassium, dans un mélange de tétrahydrofuranne et d'hexaméthyl-triamidure de phosphore, le 2,6,6-triméthyl-1-[4-hydroxy-hepta-1,6-diène-4-yl]-cyclohex-2-ène en $\alpha$-damascone.

Bien entendu, la température exerce une influence déterminante sur les temps de réaction. Le procédé, exothermique en soi, peut s'effectuer à une température voisine de la température ambiante. Des températures comprises entre 20° et 70° C environ, sont employées de préférence. A des températures inférieures, les temps de réaction deviennent trop longs, tandis qu'à des températures dépassant la limite supérieure indiquée, il y a formation de produits secondiares indésirables.

Comme indiqué plus haut, la réaction s'effectue dans un solvant organique inerte. A cet effet, on peut utiliser des solvants organiques tels des éthers, comme le tétrahydrofuranne ou l'éther diisopropylique, des amides telles la diméthylformamide ou l'hexaméthyl-triamidure de phosphore, un hydrocarbure aromatique, le benzène ou toluène par exemple, un alcool comme l'alcool éthylique ou le tert-butanol ou encore le méthylpyrrolidone ou le diméthylsulfoxyde. Des mélanges desdits solvants peuvent également être employés. Selon un mode d'exécution particulier, on utilise comme base le tert-butylate de potassium et en tant que solvant, la diméthylformamide ou un mélange de diméthylformamide et tétrahydrofuranne.

L'invention est illustrée d'une manière plus détaillée par les exemples qui suivent dans lesquels les températures sont indiquées en degrés centigrades et les abréviations ont le sens usuel dans l'art.

### Exemple 1

2,6,6-Triméthyl[but-3-ène-1-oyl]-cyclohex-2-ène (iso-$\alpha$-damascone) et
2,6,6-triméthyl[but-2-ène-1-oyl]-cyclohex-2-ène ($\alpha$-damascone)

100 g de 2,6,6-triméthyl-1-[4-hydroxy-hepta-1,6-diène-4-yl]-cyclohex-2-ène ont été ajoutés, à température ambiante et sous agitation, à une solution de 80 g de tert-butylate de potassium dans 180 ml de diméthylformamide, pendant que la température augmente à 60°. Après avoir été laissé à cette même température pendant 6 minutes, le mélange de réaction a été versé dans une solution aqueuse d'HCl [150 ml d'HCl conc./200 ml d'$H_2O$]. Une distallation par entraînement à la vapeur a fourni environ 4 l de distillat qui a été ensuite extrait avec de l'éther de pétrole (80—100°). Après les traitements usuels de séparation, séchage et évaporation des extraits organiques, on a obtenu un mélange constitué par iso-α-damascone et α-damascone (71 g).

Des rendements tout à fait analogues ont été obtenus en effectuant la réaction comme indiqué ci-dessus mais en remplaçant l'HCl aqueux par de l'acide acétique dilué. L'isomérisation complète de l'iso-α-damascone en α-damascone peut être effectuée sur le mélange obtenu au moyen d'agents d'isomérisation acides suivant le procédé décrit par exemple dans le brevet suisse no. 537 352.

## Exemple 2

### α-Damascone

Un mélange d'hydrure de potassium a été préparé en mélangeant sous azote 22 ml de KH, en dispersion à 24% dans la paraffine (0,132 M) dans 80 ml de tétrahyddrofuranne (THF) anhydre et 20 ml d'hexaméthyle triamidure de phosphore anhydre. Le mélange a été laissé sous agitation pendant 10 min puis il a été additionné goutte à goutte (15 min) de 20 g (0,086 M) de 2,6,6-triméthyl-1-[4-hydroxy-hepta-1,6-diène-4-yl]-cyclohex-2-ène. La température du mélange a été maintenue en dessous de 25°, et, à cette température, on l'a laissé sous agitation pendant 16 h, puis il a été versé sur de la glace. Par extraction à l'éther, séparation des extraits organiques et lavages successifs de ceux-ci avec une solution aqueuse 2N d'HCl, une solution de bicarbonate de sodium, et la saumure suivis de séchage sur $Na_2SO_4$ et évaporation, on a obtenu un résidu de 46,3 g. Ce produit a été dilué avec 150 ml de méthanol et la paraffine a été ainsi décantée, puis la phase méthanolique a été évaporée jusqu'à sécheresse. Le résidu a été repris avec 100 ml de THF, additionné de 15 ml d'HCl 2N et porté à reflux pendant 1 h.

Après extraction à l'éther diéthylique, les extraits organiques ont été soumis aux traitements usuels pour fournir 20,4 g d'un mélange qui, par distillation fractionnée, a fourni 9,5 g d'α-damascone ayant une pureté de 95% environ (rendement 55%).

## Exemple 3

### α-Damascone

4,5 g d'hydrure de sodium en dispersion à 80% dans la paraffine (0,15 M) ont été mélangés à 100 ml de diméthylsulfoxide et le tout a été chaufé à 65° sous agitation jusqu'à ce que le dégagement d'hydrogène cesse. Le mélange a été refroidi à 50° et a été additionné de 23,4 g (0,1 M) de 2,6,6-triméthyl-1-[4-hydroxy-hepta-1,6-diène-4-yl]-cyclohex-2-ène, puis il a été laissé à cette même température pendant 3 h sous agitation et une nuit à température ambiante, et enfin versé sur de la glace.

On a extrait à l'éther et les extraits organiques une fois réunis ont été soumis aux traitements usuels de lavage, neutralisation et séchage. Après évaporation, on a obtenu 27,6 g d'un résidu qui a été dissous dans 100 ml de THF. 15 ml d'une solution aqueuse 2N d'HCl ont été ajoutés à la solution ainsi obtenue et le tout a été maintenu à reflux pendant 1 h. Après refroidissement, le mélange réactionnel a été versé sur de la glace et extrait à l'éther diéthylique. Par les traitements habituels, on a ainsi obtenu 11,7 g d'α-damascone (rendement 61%).

## Exemple 4

### α-Damascone

23,4 g (0,1 M) de 2,6,6-triméthyl-1-[4-hydroxy-hepta-1,6-diène-4-yl]cyclohex-2-ène ont été ajoutés, sous atmosphère d'azote et avec agitation, à un mélange de 15,5 g (0,15 M) de tert-butylate de potassium dans 200 ml de diméthylformamide (DMF). La température du mélange s'était ainsi élevée à environ 40°. Après avoir été laissé sous agitation pendant 1/2 h, le mélange de réaction a été versé sur de la glace, et extrait par la suite avec de l'éther. Les extraits éthérés ont été acidifiés avec une solution 2N d'HCl et successivement lavés avec une solution aqueuse saturée de $NaHCO_3$ et d'eau, puis séchés. Par évaporation, on a ainsi obtenu 15,3 g d'un résidu qui a été repris avec 100 ml de THF. Après acidification avec 15 ml d'une solution 2N d'HCl, on a porté le tout à reflux pendant 1 h. Le mélange a été ensuite dilué avec de l'éther, lavé avec une solution de bicarbonate puis avec de l'eau jusqu'à neutralité et enfin séché pour fournir un résidu (15 g) qui, par distillation fractionnée, a fourni 12 g d'α-damascone (rendement 62%).

Exemple 5

$\beta$-Damascone

2,34 g (0,01 M) de 2,6,6-triméthyl-1-[4-hydroxy-hepta-1,6-diène-4-yl]-cyclohex-1-ène ont été ajoutés sous azote à une suspension de 1,55 g de tert-butylate de potassium (0,015 M) dans 20 ml de DMF. La température du mélange s'est ainsi élevée à 35°. Après avoir été laissé réagir pendant 15 min, le mélange de réaction a été repris à l'éther, lavé avec HCl 2N et successivement avec du $NaHCO_3$ et de l'eau jusqu'à neutralité. Par évaporation, on a ainsi obtenu un résidu qui, après dissolution dans 10 ml de THF, a été acidifié avec 1 ml d'HCl 2N et chauffé à reflux pendant 1 h. Repris enfin à l'éther, lavé et neutralisé, il a fourni un résidu (2 g) qui, par distillation, a fourni 1,55 g de $\beta$-damascone.

Exemple 6

$\beta$-Damascénone

11,6 g (0,05 M) de 2,6,6-triméthyl-1-[4-hydroxy-hepta-1,6-diène-4-yl]-cyclohexa-1,3-diène ont été traités comme indiqué à l'Exemple précédent avec 8,6 g (0,075 M) de tert-butylate de potassium dans 50 ml de DMF. La $\beta$-damascénone a été ainsi obtenue avec un rendement de 80% (7,1 g).

Exemple 7

2,5,6,6-Tétraméthyl-[but-2-ène-1-oyl]-cyclohex-2-ène

5,5 g de 2,5,6,6-tétraméthyl-1-[4-hydroxy-hepta-1,6-diène-4-yl]-cyclohex-2-ène ont été traités comme indiqué à l'Exemple 5 avec 5,8 g de tert-butylate de potassium dans 50 ml de DMF pour fournir 3,5 g d'un mélange contenant à raison de 45% le composé désiré sous la forme de son isomère trans, et de 15% de son isomère cis, ayant respectivement les formules que voici:

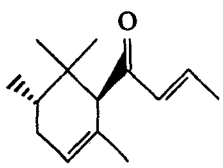

trans

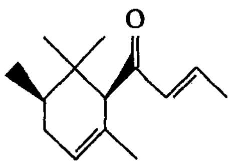

cis

Leur séparation s'effectue par chromatographie préparative en phase gazeuse.

Leurs caractères analytiques étaient les suivants:

trans:

| | |
|---|---|
| IR: | 3050, 1695, 1645, 1625, 980 et 818 $cm^{-1}$; |
| RMN ($CDCl_3$): | 0,80; 0,85; 1,55; 1,88; 2,98; 5,55; 6,3; 6,6—7,02 $\delta$ppm; |
| SM: | $M^+$ =206(22); m/e: 191(10), 163(1), 149(6), 137(30), 121(10), 109(8), 95(28), 81(12), 69(100), 57(12), 41(28). |

cis:

| | |
|---|---|
| IR: | 3045, 1690, 1665, 1625, 980 et 810 $cm^{-1}$; |
| RMN ($CDCl_3$): | 0,72; 0,87; 0,96; 1,56; 1,9; 3,18; 5,58; 6,2; 6,6—7,3 $\delta$ppm; |
| SM: | $M^+$ =206(12); m/e: 191(3), 137(18), 121(5), 109(6), 95(17), 81(5), 69(100), 57(7), 41(18). |

Les composés obtenus sont caractérisés par une odeur élégante qui s'apparente à celle développée par l'$\alpha$-damascone, la note parfumante de l'isomère trans étant plus fine, plus fruitée et fleurie que celle de l'isomère cis correspondant.

Le carbinol diallylique, utilisé comme produit de départ dans le procédé décrit ci-dessus, a été préparé conformément au schéma réactionnel que voici:

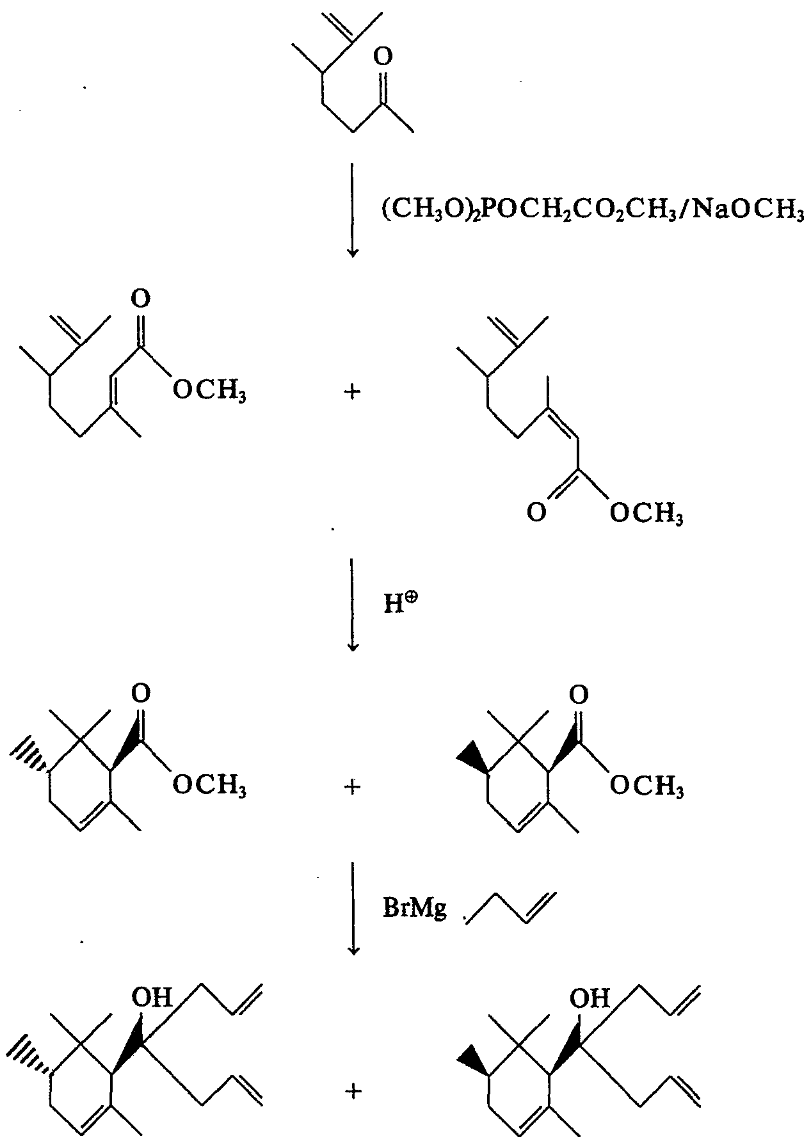


a. 42 g de 5,6-diméthyl-hept-6-ène-2-one et 60 g de triméthyl-phosphonoacétate de méthyle dans 190 ml d'éther de pétrole ont été additionnés (1¹/₂ h) sous atmosphère d'azote et agitation, de 54 g de méthylate de sodium, puis le mélange a été chauffé à reflux pendant 1¹/₂ h. Après refroidissement, dilution avec 40 ml d'eau et extraction à l'éther, on a réuni les extraits organiques et on les a soumis aux traitements usuels pour fournir 56,7 g d'un produit qui, par distallation sur colonne Vigreux à 0,05 Torr, a fourni, à 41—42°, 45 g (71%) d'un mélange de cis- et trans-2,3,6-triméthyl-octa-1,6-diénoate de méthyle.

b. 20 g du mélange d'esters obtenu suivant la lettre a. ci-dessus, ont été chauffés à reflux pendant 4 h en présence de 10 g de terre d'infusoires acide dans 100 ml de toluène. Après filtration et distallation, on a obtenu un résidu (19 g) qui, par distillation fractionnée à l'aide d'une colonne Vigreux à 0,1 Torr, a fourni 15 g d'un mélange contenant dans un rapport pondéral d'environ 55 : 30 : 15, le trans-2,5,6,6-tétraméthyl-cyclohex-2-ène-1-carboxylate de méthyle, son isomère cis et le 2,5,6,6-tétraméthyl-cyclohex-1-ène-1-carboxylate de méthyle.

Les deux esters cyclohex-2-éniques ont été séparés par chromatographie préparative en phase gazeuse.

c. 14,2 g du mélange ainsi obtenu ont été traités sous atmosphère d'azote suivant la réaction de Grignard avec 29 g de bromure d'allyle dans 100 ml d'éther anhydre en présence de 5,8 g de magnésium en copeaux et quelques cristaux de iode.

Les traitements usuels ont fourni 9,5 g d'un mélange de réaction contenant à raison de 50% le carbinol diallylique désiré.

| | |
|---|---|
| IR: | 3560, 3085, 1642, 1005, 910 et 820 $cm^{-1}$; |
| RMN ($CDCl_3$): | 0,82; 1,17; 1,82; 2,45; 4,85—6,3; 5,5 $\delta$ppm; |
| SM: | m/e: 206(1), 176(3), 153(13), 135(100), 124(24), 107(48); 93(85), 79(65), 67(48), 55(80), 41(70). |

**Revendications**

1. Procédé pour la préparation de cétones cycloaliphatiques polyinsaturées de formule

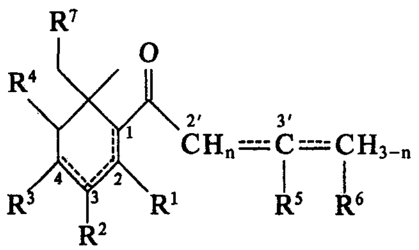

(I)

possédant une double liaison en position 2'- ou 3'- de la chaine latérale, dans laquelle l'indice n vaut 1 ou 2, et possédant en outre une double liaison isolée dans les positions 1 ou 2, ou deux doubles liaisons conjuguées dans les positions 1 et 3, comme indiqué par les pointillés et dans laquelle chacun des symboles $R^1$ à $R^7$, différents ou identiques, sert à désigner un atome d'hydrogène ou un radical alkyle inférieur, le méthyle et l'éthyle de préférence, ledit procédé étant caractérisé en ce qu'on traite au moyen d'une base forte, et dans un solvant inerte, un carbinol diallylique de formule

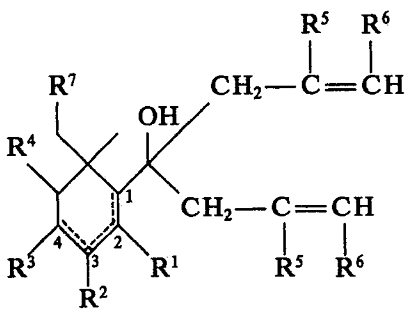

(II)

dans laquelle les symboles $R^1$ à $R^7$ ainsi que les pointillés ont le sens indiqué plus haut.

2. Procédé suivant la revendication 1, caractérisé en ce que la base forte est un hydrure, un alkoxyde ou un hydroxyde de métal alcalin.

3. Procédé suivant la revendication 2, caractérisé en ce qu'on utilise comme alkoxyde le tert-butylate de sodium ou potassium.

4. Procédé suivant la revendication 2, caractérisé en ce qu'on utilise comme hydrure, l'hydrure de sodium.

5. Procédé suivant l'une quelconque des revendications précédentes, caractérisé en ce que la réaction est effectuée dans un solvant organique inerte choisi parmi la classe des éthers, des amides ou des hydrocarbures aromatiques.

6. Procédé suivant la revendication 1, caractérisé en ce que la réaction est effectuée au moyen de tert-butylate de potassium dans le diméthylformamide et à une température comprise entre environ 20 et 70° C.

**Patentansprüche**

1. Verfahren zur Herstellung von polyungesättigten cycloaliphatischen Ketonen der Formel

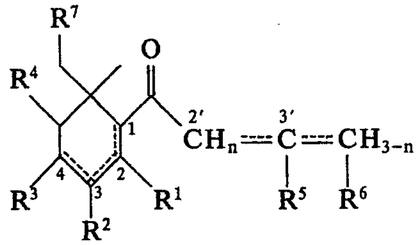

(I)

welche eine Doppelbindung in der durch die gestrichelten Linien angegeben Stellung 2′ oder 3′ der Seitenkette besitzt; der Index n 1 oder 2 ist und welche zusätzlich eine isolierte Doppelbindung in Stellung 1 oder 2 oder zwei konjugierte Doppelbindungen in Stellung 1 und 3, wie durch die gestrichelten Linien angegeben, besitzt, und worin jedes der Symbole $R^1$ bis $R^7$, welche verschieden oder gleich sein können, einen Kohlenwasserstoff oder einen niedrigen Alkylrest, vorzugsweise Methyl oder Äthyl bedeuten kann, welches dadurch gekennzeichnet ist, daß man ein Diallyl-Carbinol der Formel

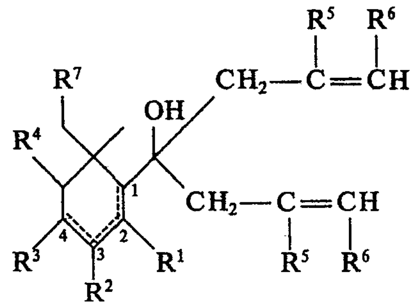

(II)

worin die Symbole $R^1$ bis $R^7$ und die gestrichelten Linien die oben angegebene Bedeutung haben, mittels einer starken Base in einem inerten Lösungsmittel, behandelt.

2. Verfahren gemäß Anspruch 1, dadurch gekennzeichnet, daß die starke Base ein Hydrid, ein Alkoxyd oder ein Alkalimetallhydroxyd ist.

3. Verfahren gemäß Anspruch 2, dadurch gekennzeichnet, daß das Alkoxyd Natrium oder Kalium tert-Butoxyd ist.

4. 2, dadurch gekennzeichnet, daß das Hydrid Natriumhydrid ist.

5. Verfahren gemäß einem der vorangehenden Ansprüche, dadurch gekennzeichnet, daß die Reaktion in einem inerten organischen Lösungsmittel, ausgewählt aus der Reihe der Äther, Amide oder aromatischen Kohlenwasserstoffe, durchgeführt wird.

6. Verfahren gemäß Anspruch 1, dadurch gekennzeichnet, daß die Reaktion unter Benützung von Kalium tert-Butoxyd in Dimethylformamid und bei einer Temperatur von zwischen ca. 20 und 70°C, durchgeführt wird.

**Claims**

1. Process for the preparation of polyunsaturated cycloaliphatic ketones of formula

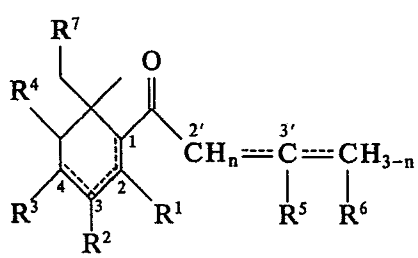

(I)

possessing a double bond in position 2′- or 3′- of the side-chain, wherein index n stands for 1 or 2, and possessing moreover an isolated double bond in position 1 or 2, ot two conjugated double bonds in positions 1 and 3 as indicated by the dotted lines and wherein each of symbols $R^1$ to $R^7$, identical or different, designates a hydrogen atom or a lower alkyl radical, preferably methyl and ethyl, said process being characterized in that a diallyl carbinol of formula

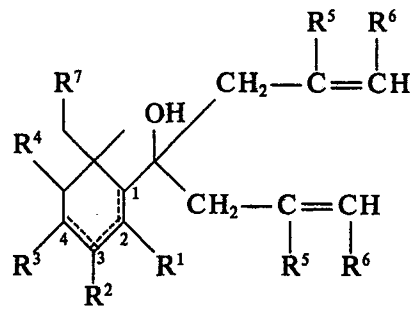

(II)

wherein symbols $R^1$ to $R^7$ and the dotted lines have the above given meaning, is treated with a strong base in an inert solvent.

2. Process according to claim 1, characterized in that the strong base is an alkali metal hydride, alkoxide or hydroxide.

3. Process according to claim 2, characterized in that the alkoxide is sodium or potassium tert-butoxide.

4. Process according to claim 2, characterized in that the hydride is sodium hydride.

5. Process according to any of the preceding claims, characterized in that the reaction is effected in an inert organic solvent chosen among ethers, amides or aromatic hydrocarbons.

6. Process according to claim 1, characterized in that the reaction is effected by using potassium tert-butoxide in dimethylformamide and at a temperature of between about 20 and 70° C.